# EUROPEAN PATENT SPECIFICATION

(11) **EP 4 587 111 B1**
(45) Date of publication and mention of the grant of the patent: **09.09.2026**
(21) Application number: 23742115.1
(22) Date of filing: 21.07.2023
(51) Int. Cl.: A61N 1/365, A61N 1/04, A61N 1/05, A61N 1/368, A61N 1/39

(54) **AN IMPLANTABLE CARDIAC RESYNCHRONIZATION THERAPY DEVICE**
IMPLANTIERBARE HERZRESYNCHRONISATIONSTHERAPIEVORRICHTUNG
DISPOSITIF DE THÉRAPIE DE RESYNCHRONISATION CARDIAQUE IMPLANTABLE

(30) Priority: 14.09.2022 EP 22195548
(43) Date of publication of application: 23.07.2025
(73) Proprietor: BIOTRONIK SE & Co. KG, 12359 Berlin (DE)
(72) Inventor: DOERR, Thomas, 12437 Berlin (DE)
(74) Representative: Biotronik Corporate Services SE
(86) International application number: PCT/EP2023/070337
(87) International publication number: WO 2024/056251

(56) References cited:
- US-A- 5 170 785
- US-A1- 2018 110 980
- US-A1- 2021 068 671
- BRÜGGEMANN THOMAS ET AL: "Tachycardia detection in modern implantable cardioverter-defibrillators", HERZSCHRITTMACHERTHERAPIE UND ELEKTROPHYSIOLOGIE, STEINKOPFF, DARMSTADT, DE, vol. 27, no. 3, 30 August 2016 (2016-08-30), pages 171 - 185, XP036117357, ISSN: 0938-7412, [retrieved on 20160830], DOI: 10.1007/S00399-016-0449-Z

## Description

The instant invention relates to an implantable cardiac resynchronization therapy device according to claim 1.

An implantable cardiac resynchronization therapy device, in short CRT device, comprises a generator including a control unit configured to control operation of the implantable cardiac resynchronization therapy device, and an arrangement of electrode leads configured for connection to the generator and for implantation in or on the ventricles for performing a resynchronization therapy for synchronizing activity of the ventricles of the patient's heart. Implantable cardiac resynchronization therapy (CRT) devices serve to establish synchronicity between ventricular contraction events. Generally, within an intact conduction system of the patient's heart the left ventricle is innervated via the so-called left bundle branch, impulses traveling equally down the left and right bundle branches from the sinoatrial node in the region of the right atrium of the heart. However, in case e.g. a so-called left bundle branch block (in short LBBB) hinders conduction along the left bundle branch, synchronous ventricular contraction of the right ventricle and the left ventricle may be disturbed. For example, in normal cardiac conduction, the septum in between the right ventricle and the left ventricle may be activated from left to right. If a left bundle branch block occurs, however, impulses may first travel via the right bundle branch to the right ventricle and then via the septum to the left ventricle, such that a conduction delay for the stimulation of the left ventricle occurs, causing asynchronous contractions of the right and left ventricles.

In a typical CRT device, three electrode leads are employed, two electrode leads being implanted such that one lead extends to the right ventricle and one lead extends to the left ventricle for providing for a stimulation and a sensing of signals on the right ventricle and on the left ventricle. In addition, a third electrode lead is implanted to reach into the right atrium, such that atrial signals may be sensed in the right atrium relating to sinoatrial activity.

There is a general desire to ease a setup for a cardiac resynchronization therapy device, limiting in particular the number of electrode leads to be implanted into or on the patient's heart.

US 5,170,785 relates to a pacing system which relates to a rate varying cardiac pacemaker (1) for electrically stimulating the heart of a pacemaker wearer. The electrical cardiogenic heart is detected on a multipolar single probe with the aid of individual electrodes (7 to 10). Bipolar electrodes in the atrium allows for detection of the intra-atrial actions (P-wave) as a first rate control signal for controlling the pacemaker in the atrially triggered ventricular pacing mode (VDD). A second rate control signal other than the P-wave correlating with patient activity is determined in parallel with the P-wave control signal.

US 2021/0068671 A1 relates to an implantable system detecting electrical signals of a human or animal heart includes a processor, a memory unit, a first detection unit for atrial activity, a second detection unit for right ventricular activity and a third detection unit for left ventricular activity.

US 2018/0110980 A1 relates to an implantable medical device including a control unit, at least a right ventricular sensing unit and/or a right atrial sensing unit, wherein the right ventricular sensing unit is connected or can be connected to a right ventricular stimulation electrode lead having at least one electrode pole and a stimulation unit that is connected or can be connected to a multipolar left ventricular stimulation electrode lead having a plurality of electrode poles.

BRUGGEMANN THOMAS ET AL: "Tachycardia detection in modem implantable cardioverter-defibrillators", HERZSCHRITTMACHERTHERAPIE UND ELEKTROPHYSIOLOGIE, STEINKOPFF, DARMSTADT, DE, vol. 27, no. 3, 30 August 2016 (2016-08-30), pages 171-185, XP036117357, relates to detection of cardiac arrhythmias and their differential diagnosis by using automatic algorithms built in implantable cardioverter-defibrillators.

It is an object of the instant invention to provide a cardiac resynchronization therapy device which allows to reduce the complexity of a cardiac resynchronization therapy device, while establishing a reliable, efficient operation. For better understanding of the invention, the present disclosure refers also to a non-claimed method of operating an implantable cardiac resynchronization therapy device.

This object is achieved by means of a cardiac resynchronization therapy device comprising the features of claim 1.

In one aspect, an implantable cardiac resynchronization therapy device comprises a generator including a control unit configured to control operation of the implantable cardiac resynchronization therapy device, and an arrangement of electrode leads configured for connection to the generator and for implantation in or on the ventricles for performing a resynchronization therapy for synchronizing activity of the ventricles of the patient's heart. At least one electrode lead of said arrangement of electrode leads comprises a first arrangement of electrode poles, arranged at a distal end of the at least one electrode lead, for sensing ventricular sense signals in one of the ventricles and for inducing a stimulation in said one of the ventricles and a second arrangement of electrode poles, arranged at a location proximal to the distal end, for sensing atrial sense signals in an atrium of the patient's heart. The control unit is configured to perform a sensing test to evaluate atrial sense signals sensed using said second arrangement of electrode poles based on at least one stability criterion and to perform, based on a result of said sensing test, an adaption routine to adapt at least one parameter for generating ventricular stimulation signals to be output using said arrangement of electrode leads. The evaluation based on said at least one stability criterion includes at least one of: evaluating whether a peak amplitude of a sensed atrial peak is larger than a first pre-defined threshold, and evaluating whether a peak amplitude of a sensed atrial peak is smaller than a second pre-defined threshold.

The cardiac resynchronization therapy device comprises an arrangement of electrode leads, one of the leads being configured to sense - in addition to providing a sensing and stimulation of ventricular signals in the ventricle, in particular the right ventricle - atrial signals, in particular in the right atrium, relating to sinoatrial activity. The electrode lead is implanted to reach into one of the ventricles of the patient's heart, in particular the right ventricle, such that a first arrangement of electrode poles in the region of a distal end of the electrode lead is placed on or in close proximity to tissue in the ventricle. At a remote location proximal to the distal end, a second arrangement of electrode poles is placed on the lead which rests in the region of the atrium, in particular the right atrium, upon implantation of the lead in the patient's heart, the second arrangement of electrode poles however being floating in that the second arrangement of electrode poles is not fixedly arranged on and connected to tissue in the atrium.

Using this electrode lead, also denoted as VDD electrode lead, signals may be sensed both in the atrium and the ventricle, in particular in the right atrium and the right ventricle. Using one electrode lead, hence, a sensing of signals in the atrium as well as in the ventricle becomes possible, such that the implantable cardiac resynchronization therapy device can use only two electrode leads, one reaching into the right ventricle and one being placed on the left ventricle to be able to provide for a stimulation on the right ventricle and on the left ventricle. An additional, third electrode for implantation such that a tip rests on tissue in the right atrium, as in common cardiac resynchronization therapy devices, hence is dispensable, the sensing of atrial signals being possible by means of one of the two ventricular electrode leads reaching through the atrium.

When using a ventricular electrode lead implanted in the ventricle, in particular the right ventricle, for sensing atrial signals, it follows that the sensing of atrial signals potentially may not be as reliable and stable as for a dedicated third electrode lead implanted specifically in the atrium, in particular the right atrium. Hence, when setting and adapting parameters for a cardiac resynchronization therapy, it must be taken care that signals are correctly and reliably sensed, such that erroneous signals do not affect the quality of the resynchronization therapy.

For this reason, the control unit is configured to perform a sensing test to evaluate atrial sense signals sensed using the second arrangement of electrode poles based on at least one stability criterion. Based on a result of the sensing test, an adaption routine is performed to adapt at least one parameter for generating ventricular stimulation signals to be output using the arrangement of electrode leads. Hence, prior to adapting parameters of the ventricular stimulation for the resynchronization therapy, a sensing test is performed in which it is evaluated whether sensed atrial signals are reliable or, instead, may potentially be erroneous. The evaluation takes place based on at least one stability criterion, which indicates, if fulfilled, that sensed atrial signals are reliable and may be trusted for adaption of parameters for a ventricular stimulation in the course of a resynchronization therapy.

The sensing test may test atrial sense signals for a potential undersensing or an oversensing. An undersensing may occur in case sensed atrial signals stemming from atrial activity are weak, for example caused by a poor reception of atrial signals by the second arrangement of electrode poles, the received signals hence not allowing for a reliable identification of atrial events. An oversensing may occur if for example signals stemming from ventricular activity, for example from a QRS waveform relating to ventricular activity, are received by the second arrangement of electrode poles, the received signals hence not relating to atrial activity.

In order to assess atrial signals for a potential undersensing, a peak amplitude of a sensed atrial peak, for example relating to a P wave indicative an atrial contraction event, is compared to a first threshold, and if it is found that the amplitude of the atrial peak is smaller than the threshold, it is concluded that an undersensing may be present. In order to assess atrial signals for a potential oversensing, a peak amplitude of a sensed atrial peak, for example relating to a P wave indicative of an atrial contraction event, is compared to a second threshold, and if it is found that the amplitude of the atrial peak is larger than the second threshold, it is concluded that an oversensing may be present. In both cases sensed signals may be identified as unreliable, such that the sensing test may output a non-successful result.

In one embodiment, the control unit is configured to perform the adaption routine if and only if the sensing test is successful. The sensing test herein is deemed to be successful if the at least one stability criterion is fulfilled.

In one embodiment, the control unit is configured to inhibit the performing of the adaption routine if the sensing test is not successful. Hence, if the sensing test is not successful, i.e., the at least one stability criterion is not fulfilled, an adaption of parameters for the ventricular stimulation is prevented.

For example, if the sensing test is not successful, the carrying out of the adaption routine is inhibited for a predefined number of cardiac cycles, for example for a number of cardiac cycles in between 10 to 1000, for example 50 to 500. In another embodiment, if the sensing test is not successful, the carrying out of the adaption routine is inhibited for a predefined time, for example a time in between 10 seconds to 1000 seconds, for example 50 to 500 seconds. In yet another embodiment, if the sensing test is not successful, the carrying out of the adaption routine is inhibited permanently until an examination by a physician is carried out, the adaption routine being then activated again e.g. by the physician.

The result of the sensing test and/or the inhibiting of the adaption routine may be communicated to an external device, e.g., in a home monitoring system.

In one embodiment, said evaluation based on said at least one stability criterion includes at least one of: evaluating whether a sensed atrial interval is within a predefined first range, evaluating whether a change in a sensed atrial interval over a predefined number of cardiac cycles is within a predefined first limit, evaluating whether a peak amplitude of a sensed atrial peak is within a predefined second range, and evaluating whether a change of a peak amplitude of a sensed atrial peak over a predefined number of cardiac cycles is within a predefined second limit.

An atrial interval may be measured as a so-called PP interval, which is the time distance between successive peaks relating to atrial P waves. A peak amplitude may be measured according to the amplitude of a P wave.

Within the stability criterion assessment, it is determined for example whether an atrial interval is plausible by checking whether the atrial interval falls into a predefined (first) range, the range being limited by an upper bound and a lower bound and being indicative of a normal, physiologically plausible range of a PP interval.

Alternatively or in addition, a change in the atrial interval over a number of cardiac cycles may be monitored, wherein the atrial interval as sensed and determined according to atrial sense signals is found to be stable if the change in the atrial interval over the cardiac cycles is smaller than a predefined (first) limit.

Yet alternatively or in addition, a peak amplitude is assessed in that it is determined whether the peak amplitude falls into a predefined (second) range, hence assessing whether a sensed peak amplitude value is plausible in that it may relate to an actual physiological event.

Yet alternatively or in addition, a change in peak amplitude over a predefined number of cardiac cycles is observed, wherein the peak amplitude is found to be stable if the change in the peak amplitude over the cardiac cycles is smaller than a predefined (second) limit.

Further criterion's may be employed, for example a so-called short-long-short criterion relating to a QRS far field recognition.

The sensing test may evaluate sensed signals for example over a sequence of at least one cardiac cycle, preferably over at least two cardiac cycles, while measuring intrinsic atrial activity.

In one embodiment, the control unit is configured, within the adaption routine, to measure an intrinsic atrial-ventricular delay based on an atrial sense signal and a ventricular sense signal and to adapt at least one parameter for a subsequent stimulation based on a measured intrinsic atrial-ventricular delay. For example, the control unit may be configured to set an atrial-ventricular delay value to be used in a stimulation, denoted herein as paced atrial-ventricular delay, based on the measured ventricular atrial-ventricular delay and to generate ventricular stimulation signals to be output using the arrangement of electrode leads based on the paced atrial-ventricular delay. Based on a measured intrinsic atrial-ventricular delay as obtained during the adaption routine, a value for a paced atrial-ventricular delay is set, for example by using the measured intrinsic atrial-ventricular delay modified by a predefined factor or by adding or subtracting a predefined constant value to/from the measured intrinsic atrial-ventricular delay. Based on the paced atrial-ventricular delay value, hence, ventricular stimulation signals in the context of the resynchronization therapy are generated in order to achieve a synchronous ventricular activity.

Within the adaption routine, the intrinsic atrial-ventricular delay may for example be measured by setting, during the regular stimulation process, a paced atrial-ventricular delay as used for the stimulation to a large value in order to ensure that intrinsic events occur prior to stimulation events. By then measuring atrial signals and subsequent ventricular signals (in one or both ventricles), an atrial-ventricular delay value indicative of a conduction delay in the conduction system between the atrium and the right and/or left ventricle may be determined.

As the adaption routine is carried out based on the prior sensing test, the paced atrial-ventricular delay is determined and used only if it is found, in the sensing test, that reliable atrial sense signals are available and may be used for reliably measuring an intrinsic atrial-ventricular delay.

In one embodiment, the control unit is configured to generate ventricular stimulation signals either for a bi-ventricular simulation or for a stimulation in only one of the ventricles based on the measured intrinsic atrial-ventricular delay. Based on the measured intrinsic atrial-ventricular delay the control unit may conclude that a bi-ventricular simulation or a stimulation in only one of the ventricles, for example the left ventricle, shall be carried out. Based on a value for the intrinsic atrial-ventricular delay, hence, operation of the cardiac resynchronization therapy device for performing a cardiac resynchronization therapy is adapted.

In one embodiment, the control unit is configured, within the adaption routine, to measure a first intrinsic atrial-ventricular delay based on an atrial sense signal and a first ventricular sense signal sensed in one of the ventricles and a second intrinsic atrial-ventricular delay based on an atrial sense signal and a second ventricular sense signal sensed in the other of the ventricles. Hence, different intrinsic atrial-ventricular delay values are determined, relating to an atrial-ventricular delay in the conduction system in between the atrium and the right ventricle and between the atrium and the left ventricle.

The different intrinsic atrial-ventricular delay values may be set in relation to one another in order to adapt the operation of a stimulation process.

For example, the control unit may be configured to adapt said at least one parameter for generating stimulation signals based on a comparison of the first intrinsic atrial-ventricular delay and the second intrinsic atrial-ventricular delay. The first intrinsic atrial-ventricular delay value may for example relate to a delay between an atrial signal and a right ventricular signal, whereas the second intrinsic atrial-ventricular delay value may relate to a delay between and atrial signal and a left ventricular signal. If the first intrinsic atrial-ventricular delay is found to be larger than the second intrinsic atrial-ventricular delay, for example a bi-ventricular pacing may be carried out, for example using a preprogrammed paced atrial-ventricular delay value. If the first intrinsic atrial-ventricular delay and the second intrinsic atrial-ventricular delay are found to be (approximately) equal, a bi-ventricular pacing may be employed, by using a paced atrial-ventricular delay value which is set according to the measured (first and/or second) intrinsic atrial-ventricular delay. If the first intrinsic atrial-ventricular delay is found to be smaller than the second intrinsic atrial-ventricular delay, for example a stimulation only in the left ventricle may be employed by using a paced atrial-ventricular delay value which is set according to a measured (first and/or second) intrinsic atrial-ventricular delay value.

In one embodiment, the control unit is configured to perform the sensing test cyclically by employing a cycle length corresponding to a predefined number of cardiac cycles, for example a number of cardiac cycles in a range between 5 to 100 cardiac cycles. The sensing test herein may be integrated for example in the cyclic adaption routine, which for example is cyclically performed in order to automatically adapt and set parameters of the stimulation for the synchronization therapy. For example, each time the adaption routine is carried out the sensing test is performed prior to the adaption routine.

In one embodiment, the implantable cardiac resynchronization therapy device is a CRT-D device. A CRT-D device includes, in addition to the resynchronization function, a defibrillation function analogous to an ICD device.

In one embodiment, the implantable cardiac resynchronization therapy device is a CRT-P device. A CRT-P device includes, in addition to the resynchronization function, a cardiac pacemaker function for pacing cardiac activity.

In another aspect, a method for operating an implantable cardiac resynchronization therapy device comprises: providing a generator including a control unit configured to control operation of the implantable cardiac resynchronization therapy device; and providing an arrangement of electrode leads configured for connection to the generator and for implantation in or on the ventricles for performing a re-synchronization therapy for synchronizing activity of the ventricles of the patient's heart. The method further includes: sensing ventricular sense signals in one of the ventricles and inducing a stimulation in said one of the ventricles using a first arrangement of electrode poles arranged at a distal end of at least one electrode lead of said arrangement of electrode leads of the at least one electrode lead; sensing atrial sense signals in an atrium of the patient's heart using a second arrangement of electrode poles arranged at a location proximal to the distal end of said at least one electrode lead; performing, using the control unit, a sensing test to evaluate atrial sense signals sensed using said second arrangement of electrode poles based on at least one stability criterion; and performing, using the control unit and based on a result of said sensing test, an adaption routine to adapt at least one parameter for generating ventricular stimulation signals to be output using said arrangement of electrode leads. The evaluation based on said at least one stability criterion includes at least one of: evaluating whether a peak amplitude of a sensed atrial peak is larger than a first pre-defined threshold, and evaluating whether a peak amplitude of a sensed atrial peak is smaller than a second pre-defined threshold.

The advantages and advantageous embodiments described above for the cardiac resynchronization therapy device equally apply also to the method such that it shall be referred to the above in this respect.

The idea underlying the invention shall subsequently be described in more detail with reference to the embodiments shown in the figures. Herein:
- Fig. 1: shows a schematic drawing of a cardiac resynchronization therapy device in an implanted state, comprising electrode leads implanted in a patient's heart;
- Fig. 2: shows a schematic flow diagram of an adaption routine with a prior sensing test; and
- Fig. 3: shows a schematic flow diagram of a sensing test.

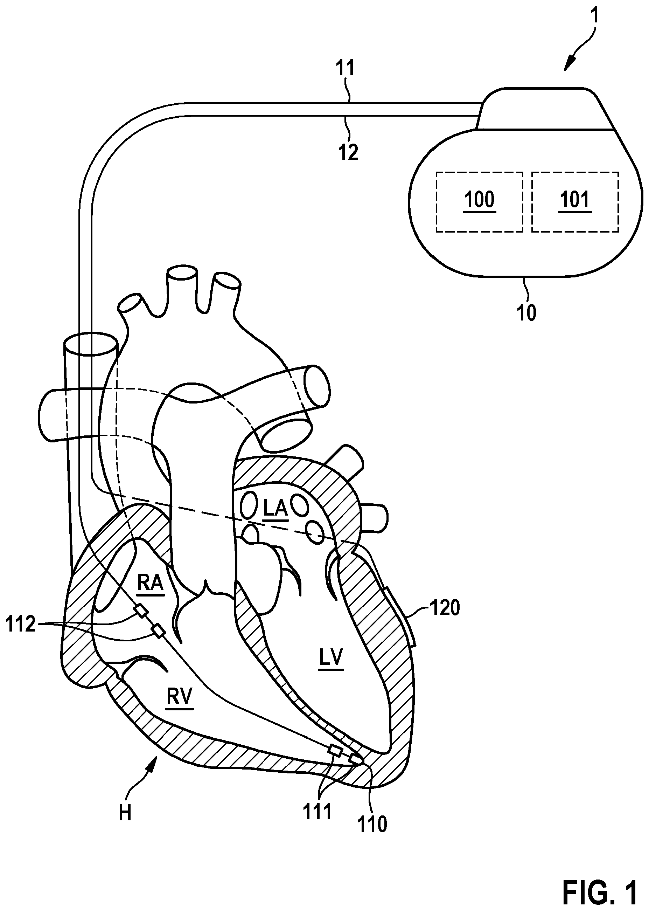

Fig. 1 shows, in a schematic drawing, a cardiac resynchronization therapy (CRT) device 1 comprising a generator 10 to which a pair of electrode leads 11, 12 are connected.

The generator 10 comprises a control unit 100 and further functional components, such as for example an energy storage device 101, for example a battery. The control unit 100 is configured to generate stimulation signals in order to perform a cardiac resynchronization therapy for stimulating ventricular activity to cause synchronous contraction events in the right ventricle RV and left ventricle LV of a patient's heart H.

The generator 10 is designed for e.g. a subcutaneous implantation and rests, in an implanted state, outside of the patient's heart H. The electrode leads 11, 12 herein extend from the generator 10 into the patient's heart H, for example through the subclavian vein into the right atrium RA and from the right atrium RA to the right ventricle RV (lead 11) or from the right atrium RA through the coronary venous system to an outer wall of the left ventricle LV (lead 12.

In an implanted state, a distal tip 110 of the electrode lead 11 is implanted in the right ventricle RV such that the tip 110 rests on tissue within the right ventricle RV to provide for a stimulation on the right ventricle RV. A distal tip 120 of the electrode lead 12, in turn, is implanted such that it rests on the outer wall of the left ventricle LV for providing for a stimulation on the left ventricle LV.

In the course of a cardiac resynchronization therapy, stimulation signals are generally generated in accordance with sense signals indicative of atrial-ventricular delays in the conduction system between the right atrium RA and the right ventricle RV and the right atrium RA and the left ventricle LV in order to establish a synchronicity between ventricular contraction events in the right ventricle RV and the left ventricle LV.

In the cardiac resynchronization therapy device 1 as shown in Fig. 1, herein, only two electrode leads 11, 12 are used which are implanted to reach into the right ventricle RV (electrode lead 11) and the left ventricle LV (electrode lead 12). Hence, no dedicated atrial sense electrode is employed which is implanted in the right atrium and provides for a dedicated sensing of atrial signals in the right atrium RA.

Instead, the electrode lead 11 implanted in the right ventricle RV functions as a so-called VDD electrode which comprises a first electrode pole arrangement 111 at the distal tip 110 of the electrode lead 11 and an additional, second arrangement of electrode poles 112 which floatingly is arranged, in an implanted state of the electrode lead 11, in the right atrium RA.

As the arrangement of electrode poles 112 is not placed immediately on tissue in the right atrium RA, no direct coupling between tissue and the electrode poles 112, for example a pair of ring electrodes placed on the lead body of the electrode lead 11, is established such that atrial signals may not be picked up as reliably and as strong as with a dedicated atrial sense electrode. Rather, the quality of sensed atrial signals as sensed by means of the electrode poles 112 may depend on the floating placement of the electrode lead 11 in the right atrium RA.

For this reason, the control unit 100 of the generator 10 of the cardiac resynchronization therapy device 1 is configured to carry out a sensing test in order to establish whether sensed atrial signals as sensed by means of the arrangement of electrode poles 112 are reliable and may be used to adapt parameters for providing for a stimulation action in the course of the cardiac resynchronization therapy.

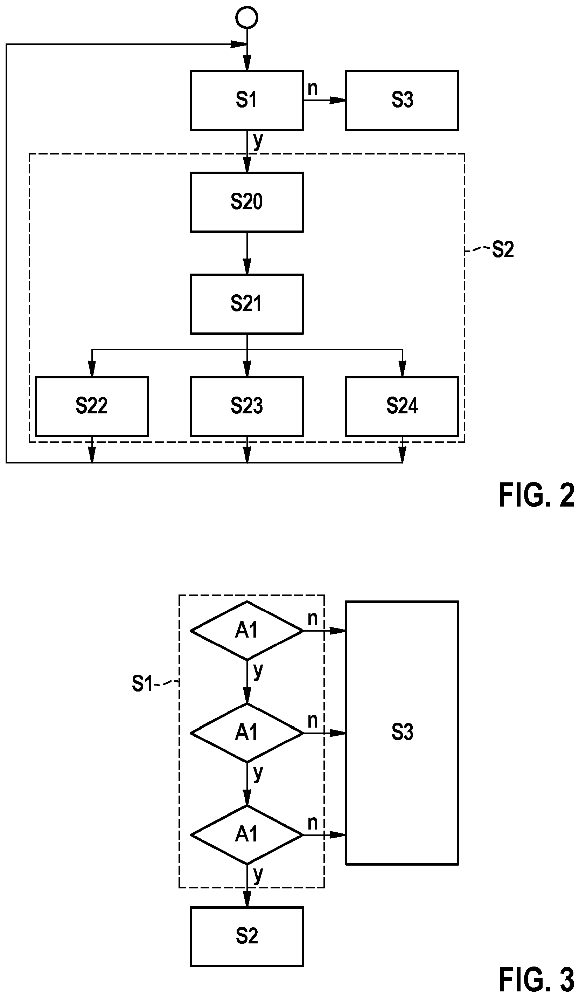

Referring now to Fig. 2, within a cardiac resynchronization therapy, an adaption routine S2 may be carried out in order to adapt certain parameters for a ventricular stimulation in order to establish a synchronicity of ventricular contraction events. Prior to carrying out the adaption routine S2, herein, a sensing test S1 is performed, in the course of which it is examined whether sensed atrial signals as received by means of the arrangement of electrode poles 112 are stable such that it may be assumed that the sensed atrial signals reliably indicate atrial contraction events.

Herein, only if the sensing test S1 yields a successful result (y), the adaption routine S2 is started, and parameters of a stimulation process are set and adapted within the adaption routine S2. If, in contrast, the sensing test S1 yields a negative result (n), the adaption routine S2 may be inhibited for a predefined number of cardiac cycles, for a predefined time, or until an examination by a physician is carried out.

Referring now to Fig. 3, in the sensing test S1 it may in particular be checked whether sensed atrial signals are plausible in that they likely originate from physiological atrial activity.

For example, in one embodiment, it may be checked within the sensing test S1 whether an atrial interval falls into a predefined range (step A1), such that it is checked whether a heart rate as indicated by the atrial interval is plausible. For example, the atrial interval may be determined as a time interval between successive atrial peaks, in particular relating to consecutive P waves relating to atrial contraction events.

If the first check for the atrial interval in step A1 yields a positive result (y), it in a further step A2 is checked whether a change in the atrial interval over a predefined number of cardiac cycles is smaller than a predefined limit, such that the atrial interval can be assumed to be stable over a number of cardiac cycles. The cardiac cycles not necessarily are consecutive. Rather, it for example in each sensing test may be determined whether the atrial interval has changed with respect to a prior sensing test by a margin smaller than the predefined limit.

If the second check in step A2 yields a positive result (y), it in a further step A3 is checked whether a peak amplitude is stable. For this, it for example may be checked whether the peak amplitude falls into a predefined range, and/or whether a change in the peak amplitude over a number of cardiac cycles is smaller than a predefined limit.

If the third step A3 also yields a positive result (y), the sensing test is concluded to be successful, and the adaption routine S2 is started.

If in any step A1 to A3 the check yields a negative result (n), the adaption routine S2 is inhibited (step S3), such that no adaption of parameters of the stimulation routine is carried out, but rather prior values or preprogrammed values may be used for the cardiac resynchronization therapy.

The sensing test S1 may include additional checks, or other checks than the ones illustrated according to Fig. 3. Also, not all of the steps necessarily need to yield a positive result, but the sensing test S1 may be assumed to yield a positive result already if one or a group of the steps are positive.

Referring now back to Fig. 2, in the automatic adaption routine S2 parameters for the cardiac resynchronization therapy are automatically set such that an optimized resynchronization therapy by generating ventricular stimulation signals may be performed.

When the sensing test S1 has been successfully performed (y), the adaption routine S2 is entered. At the start of the adaption routine S2 a long atrial-ventricular delay in the pacing algorithm for the cardiac resynchronization therapy is set (step S20). By setting a long paced atrial-ventricular delay it is achieved that, likely, intrinsic events occur prior to stimulation events and may be measured within the adaption routine S2 by making use of electrode poles 111, 112 on the electrode leads 11, 12.

Once the atrial-ventricular delay has been set to a long value (step S20), such that the regular stimulation of the resynchronization therapy is temporarily inhibited, values for an intrinsic atrial-ventricular delay indicative of a conduction in between the right atrium RA and the right ventricle RV and of a conduction between the right atrium RA and left ventricle LV are measured to obtain a measured atrial-right ventricular (A-RV) delay value and a measured atrial-left ventricular (A-LV) delay value (step S21).

Based on the measured atrial-ventricular delay values, now, operation of the stimulation in the synchronization therapy is automatically adapted.

For example, for adapting parameters of the synchronization therapy, the values of the atrial-right ventricular (A-RV) delay and the atrial-left ventricular (A-LV) delay may be compared to each other.

If it is found that the atrial-right ventricular (A-RV) delay is smaller than the atrial-left ventricular (A-LV) delay, for example a pacing may take place only in the left ventricle LV. In addition, an optimized, paced atrial-ventricular delay value computed based on the measured atrial-ventricular delay values may be used in the algorithm for generating stimulation signals (step S22).

If it is found that the atrial-right ventricular (A-RV) delay approximately equals the atrial-left ventricular (A-LV) delay, a bi-ventricular pacing by stimulating the right ventricle RV and left ventricle LV may be employed, while using an optimized paced atrial-ventricular delay value as computed based on the measured atrial-ventricular delay values (step S23).

If it is found that the atrial-right ventricular (A-RV) delay is larger than the atrial-left ventricular (A-LV) delay, for example a bi-ventricular stimulation may be employed, by using a preprogrammed value for the paced atrial-ventricular delay, hence not computing a paced atrial-ventricular delay value based on measured atrial-ventricular delay values (step S24).

The adaption routine S2, with the prior sensing test S1, may be cyclically carried out, for example at a cyclic interval of a predefined number of cardiac cycles, for example in a range between 10 to 1000 cardiac cycles, for example 20 to 100 cardiac cycles. In cardiac cycles subsequent to the adaption routine S2, parameters as set during the adaption routine S2 may be used for the stimulation in the context of the cardiac resynchronization therapy.

If the sensing test S1 does not yield a positive result and it hence is found that no atrial signals are reliably sensed, the adaption routine S2 is inhibited, for example for a predefined number of cardiac cycles or for a predefined time, after which a sensing test S1 is carried out anew. In another embodiment, the adaption routine S2 may be inhibited until an examination is carried out by a physician, who may then activate the adaption routine anew.

The idea underlying the invention is not limited to the embodiments described above, but may be implemented in entirely different embodiments.

In particular, other parameters than the ones described herein may be adapted by means of an automatic adaption routine.

Further, a stability of atrial sense signals may be assessed based on other criterions than the ones described in this text.

### List of reference numerals

- 1: CRT device
- 10: Generator
- 100: Control unit
- 101: Energy storage unit
- 11: Electrode lead
- 110: Distal end
- 111: Electrode poles
- 112: Electrode poles
- 12: Electrode lead
- 120: Distal end
- H: Heart
- LA: Left atrium
- LV: Left ventricle
- RA: Right atrium
- RV: Right ventricle
- S1, S2, S3: Algorithm steps
- S20-S24: Algorithm steps

## Claims

1. An implantable cardiac resynchronization therapy device (1), comprising:
a generator (10) including a control unit (100) configured to control operation of the implantable cardiac resynchronization therapy device (1), and
an arrangement of electrode leads (11, 12) configured for connection to the generator (10) and for implantation in or on the ventricles (LV, RV) for performing a resynchronization therapy for synchronizing activity of the ventricles (LV, RV) of the patient's heart (H),
**wherein** at least one electrode lead of said arrangement of electrode leads (11, 12) comprises a first arrangement of electrode poles (111), arranged at a distal end (110, 120) of the at least one electrode lead, for sensing ventricular sense signals in one of the ventricles and for inducing a stimulation in said one of the ventricles and a second arrangement of electrode poles (112), arranged at a location proximal to the distal end (110, 120), for sensing atrial sense signals in an atrium of the patient's heart (H),
wherein the control unit (100) is configured to perform a sensing test (S1) to evaluate atrial sense signals sensed using said second arrangement of electrode poles (112) based on at least one stability criterion and to perform, based on a result of said sensing test (S1), an adaption routine (S2) to adapt at least one parameter for generating ventricular stimulation signals to be output using said arrangement of electrode leads (11, 12),
**characterized in that** said evaluation based on said at least one stability criterion includes at least one of: evaluating whether a peak amplitude of a sensed atrial peak is larger than a first pre-defined threshold, and evaluating whether a peak amplitude of a sensed atrial peak is smaller than a second pre-defined threshold.

2. The implantable cardiac resynchronization therapy device (1) according to claim 1, **characterized in that** the control unit (100) is configured to perform said adaption routine (S2) if and only if the sensing test (S1) is successful.

3. The implantable cardiac resynchronization therapy device (1) according to claim 1 or 2, **characterized in that** the control unit (100) is configured to inhibit the performing of the adaption routine (S2) if the sensing test (S1) is not successful.

4. The implantable cardiac resynchronization therapy device (1) according to one of the preceding claims, **characterized in that** said evaluation based on said at least one stability criterion includes at least one of: evaluating whether a sensed atrial interval is within a pre-defined first range, evaluating whether a change in a sensed atrial interval over a pre-defined number of cardiac cycles is within a predefined first limit, evaluating whether a peak amplitude of a sensed atrial peak is within a pre-defined second range, and evaluating whether a change of a peak amplitude of a sensed atrial peak over a pre-defined number of cardiac cycles is within a pre-defined second limit.

5. The implantable cardiac resynchronization therapy device (1) according to one of the preceding claims, **characterized in that** the control unit (100) is configured, within said adaption routine (S2), to measure an intrinsic atrial-ventricular delay based on an atrial sense signal and a ventricular sense signal and to adapt at least one parameter based on a measured intrinsic atrial-ventricular delay.

6. The implantable cardiac resynchronization therapy device (1) according to claim 5, **characterized in that** the control unit (100) is configured to set a paced atrial-ventricular delay based on said measured intrinsic atrial-ventricular delay and to generate ventricular stimulation signals to be output using said arrangement of electrode leads (11, 12) based on said paced atrial-ventricular delay.

7. The implantable cardiac resynchronization therapy device (1) according to claim 5 or
6, **characterized in that** the control unit (100) is configured to generate ventricular stimulation signals either for a bi-ventricular stimulation or for a stimulation in only one of the ventricles (LV, RV) based on said measured intrinsic atrial-ventricular delay.

8. The implantable cardiac resynchronization therapy device (1) according to one of the preceding claims, **characterized in that** the control unit (100) is configured, within said adaption routine (S2), to measure a first intrinsic atrial-ventricular delay based on an atrial sense signal and a first ventricular sense signal sensed in one of the ventricles (LV, RV) and a second intrinsic atrial-ventricular delay based on an atrial sense signal and a second ventricular sense signal sensed in the other of the ventricles (LV, RV).

9. The implantable cardiac resynchronization therapy device (1) according to claim 8, **characterized in that** the control unit (100) is configured to adapt said at least one parameter based on a comparison of the first atrial-ventricular delay and the second atrial-ventricular delay.

10. The implantable cardiac resynchronization therapy device (1) according to one of the preceding claims, **characterized in that** the control unit (100) is configured to perform said sensing test (S1) cyclically employing a cycle length corresponding to a pre-defined number of cardiac cycles.

11. The implantable cardiac resynchronization therapy device (1) according to one of the preceding claims, **characterized in that** implantable cardiac resynchronization therapy device (1) is a CRT-D device, wherein the CRT-D device includes, in addition to the resynchronization function, a defibrillation function analogous to an ICD device.

12. The implantable cardiac resynchronization therapy device (1) according to one of the preceding claims, **characterized in that** implantable cardiac resynchronization therapy device (1) is a CRT-P device, wherein the CRT-P device includes, in addition to the resynchronization function, a cardiac pacemaker function for pacing cardiac activity.

## Patentansprüche

1. Implantierbare Vorrichtung zur kardialen Resynchronisationstherapie (1), umfassend:
einen Generator (10) mit einer Steuereinheit (100), die dazu ausgelegt ist, den Betrieb der implantierbaren Vorrichtung zur kardialen Resynchronisationstherapie (1) zu steuern, und
eine Anordnung von Elektrodenleitungen (11, 12), die zum Anschluss an den Generator (10) und zur Implantation in oder an den Ventrikeln (LV, RV) konfiguriert sind, um eine Resynchronisationstherapie zur Synchronisierung der Aktivität der Ventrikel (LV, RV) des Herzens (H) des Patienten durchzuführen,
**wobei** mindestens eine Elektrodenleitung der genannten Anordnung von Elektrodenleitungen (11, 12) eine erste Anordnung von Elektrodenpolen (111) umfasst, die an einem distalen Ende (110, 120) der mindestens einen Elektrodenleitung angeordnet ist, um ventrikuläre Erfassungssignale in einem der Ventrikel zu erfassen und eine Stimulation in dem genannten einen der Ventrikel auszulösen, sowie eine zweite Anordnung von Elektrodenpolen (112), die an einer Stelle proximal zum distalen Ende (110, 120) angeordnet ist, zum Erfassen von atrialen Erfassungssignalen in einem Vorhof des Herzens (H) des Patienten,
wobei die Steuereinheit (100) so konfiguriert ist, dass sie einen Erfassungstest (S1) durchführt, um die unter Verwendung der zweiten Anordnung von Elektrodenpolen (112) erfassten atrialen Erfassungssignale auf der Grundlage mindestens eines Stabilitätskriteriums zu bewerten, und auf der Grundlage eines Ergebnisses des Erfassungstests (S1) eine Anpassungsroutine (S2) durchführt, um mindestens einen Parameter zur Erzeugung ventrikulärer Stimulationssignale anzupassen, die unter Verwendung der Anordnung von Elektrodenleitungen (11, 12) ausgegeben werden sollen,
**dadurch gekennzeichnet, dass** die Bewertung auf der Grundlage des mindestens einen Stabilitätskriteriums mindestens eines der folgenden Elemente umfasst: Bewerten, ob eine Spitzenamplitude einer erfassten atrialen Spitze größer als ein erster vordefinierter Schwellenwert ist, und Bewerten, ob eine Spitzenamplitude einer erfassten atrialen Spitze kleiner als ein zweiter vordefinierter Schwellenwert ist.

2. Implantierbare Vorrichtung zur kardialen Resynchronisationstherapie (1) nach Anspruch 1, **dadurch gekennzeichnet, dass** die Steuereinheit (100) so konfiguriert ist, dass sie die Anpassungsroutine (S2) genau dann ausführt, wenn der Erfassungstest (S1) erfolgreich ist.

3. Implantierbare Vorrichtung zur kardialen Resynchronisationstherapie (1) nach Anspruch 1 oder 2, **dadurch gekennzeichnet, dass** die Steuereinheit (100) so konfiguriert ist, dass sie die Ausführung der Anpassungsroutine (S2) unterbindet, wenn der Erfassungstest (S1) nicht erfolgreich ist.

4. Implantierbare Vorrichtung zur kardialen Resynchronisationstherapie (1) nach einem der vorstehenden Ansprüche, **dadurch gekennzeichnet, dass** die auf dem mindestens einen Stabilitätskriterium basierende Bewertung mindestens eines der folgenden Elemente umfasst: die Bewertung, ob ein erfasster Vorhofintervall innerhalb eines vordefinierten ersten Bereichs liegt, die Bewertung, ob eine Änderung eines erfassten Vorhofintervalls über eine vordefinierte Anzahl von Herzzyklen innerhalb einer vordefinierten ersten Grenze liegt, die Bewertung, ob eine Spitzenamplitude eines erfassten Vorhofspitzenwerts innerhalb eines vordefinierten zweiten Bereichs liegt, und die Bewertung, ob eine Änderung der Spitzenamplitude eines erfassten Vorhofspitzenwerts über eine vordefinierte Anzahl von Herzzyklen innerhalb einer vordefinierten zweiten Grenze liegt.

5. Implantierbare Vorrichtung zur kardialen Resynchronisationstherapie (1) nach einem der vorstehenden Ansprüche, **dadurch gekennzeichnet, dass** die Steuereinheit (100) so konfiguriert ist, dass sie innerhalb der Anpassungsroutine (S2) eine intrinsische atrioventrikuläre Verzögerung auf der Grundlage eines atrialen Erfassungssignals und eines ventrikulären Erfassungssignals misst und mindestens einen Parameter auf der Grundlage einer gemessenen intrinsischen atrioventrikulären Verzögerung anpasst.

6. Implantierbare Vorrichtung zur kardialen Resynchronisationstherapie (1) nach Anspruch 5, **dadurch gekennzeichnet, dass** die Steuereinheit (100) so konfiguriert ist, dass sie eine stimulierte atrioventrikuläre Verzögerung auf der Grundlage der gemessenen intrinsischen atrioventrikulären Verzögerung einstellt und ventrikuläre Stimulationssignale erzeugt, die unter Verwendung der Anordnung von Elektrodenleitungen (11, 12) auf der Grundlage der stimulierten atrioventrikulären Verzögerung ausgegeben werden.

7. Implantierbare Vorrichtung zur kardialen Resynchronisationstherapie (1) nach Anspruch 5 oder 6, **dadurch gekennzeichnet, dass** die Steuereinheit (100) so konfiguriert ist, dass sie ventrikuläre Stimulationssignale entweder für eine biventrikuläre Stimulation oder für eine Stimulation in nur einem der Ventrikel (LV, RV) auf der Grundlage der gemessenen intrinsischen atrioventrikulären Verzögerung erzeugt.

8. Implantierbare Vorrichtung zur kardialen Resynchronisationstherapie (1) nach einem der vorstehenden Ansprüche, **dadurch gekennzeichnet, dass** die Steuereinheit (100) so konfiguriert ist, dass sie innerhalb der Anpassungsroutine (S2) eine erste intrinsische atrioventrikuläre Verzögerung auf der Grundlage eines atrialen Erfassungssignals und eines ersten ventrikulären Erfassungssignals, das in einem der Ventrikel (LV, RV) erfasst wird, sowie eine zweite intrinsische atrioventrikuläre - ventrikuläre Verzögerung auf der Grundlage eines atrialen Erfassungssignals und eines zweiten ventrikulären Erfassungssignals, das in dem anderen der Ventrikel (LV, RV) erfasst wurde, zu messen.

9. Implantierbare Vorrichtung zur kardialen Resynchronisationstherapie (1) nach Anspruch 8, **dadurch gekennzeichnet, dass** die Steuereinheit (100) so konfiguriert ist, dass sie den mindestens einen Parameter auf der Grundlage eines Vergleichs der ersten atrioventrikulären Verzögerung und der zweiten atrioventrikulären Verzögerung anpasst.

10. Implantierbare Vorrichtung zur kardialen Resynchronisationstherapie (1) nach einem der vorstehenden Ansprüche, **dadurch gekennzeichnet, dass** die Steuereinheit (100) so konfiguriert ist, dass sie den Erfassungstest (S1) zyklisch unter Verwendung einer Zykluslänge durchführt, die einer vordefinierten Anzahl von Herzzyklen entspricht.

11. Implantierbare Vorrichtung zur kardialen Resynchronisationstherapie (1) nach einem der vorstehenden Ansprüche, **dadurch gekennzeichnet, dass** die implantierbare Herzresynchronisationstherapievorrichtung (1) eine CRT-D-Vorrichtung ist, wobei die CRT-D-Vorrichtung zusätzlich zur Resynchronisationsfunktion eine Defibrillationsfunktion analog zu einer ICD-Vorrichtung umfasst.

12. Implantierbare Vorrichtung zur kardialen Resynchronisationstherapie (1) nach einem der vorstehenden Ansprüche, **dadurch gekennzeichnet, dass** das implantierbare Herzresynchronisationstherapiegerät (1) ein CRT-P-Gerät ist, wobei das CRT-P-Gerät zusätzlich zur Resynchronisationsfunktion eine Herzschrittmacherfunktion zur Stimulation der Herzaktivität umfasst.

## Revendications

1. Dispositif de thérapie de resynchronisation cardiaque implantable (1), comprenant :
un générateur (10) incluant une unité de commande (100) configurée pour commander le fonctionnement du dispositif de thérapie de resynchronisation cardiaque implantable (1), et
un agencement de câbles d'électrode (11, 12) configuré pour connexion au générateur (10) et pour implantation dans ou sur les ventricules (LV, RV) afin de réaliser une thérapie de resynchronisation pour synchroniser l'activité des ventricules (LV, RV) du cœur du patient (H),
**dans lequel** au moins un câble d'électrode dudit agencement de câbles d'électrode (11, 12) comprend un premier agencement de pôles d'électrode (111), agencés au niveau d'une extrémité distale (110, 120) de l'au moins un câble d'électrode, pour détecter les signaux de détection ventriculaire dans l'un des ventricules et pour induire une stimulation dans ledit un des ventricules et un second agencement de pôles d'électrode (112), agencés au niveau d'un emplacement proximal vis-à-vis de l'extrémité distale (110, 120), pour détecter les signaux de détection atriale dans un atrium du cœur du patient (H),
dans lequel l'unité de commande (100) est configurée pour réaliser un test de détection (S1) afin d'évaluer les signaux de détection atriale détectés en utilisant ledit second agencement de pôles d'électrode (112) en se basant sur au moins un critère de stabilité et pour réaliser, en se basant sur un résultat dudit test de détection (S1), une routine d'adaptation (S2) afin d'adapter au moins un paramètre pour générer des signaux de stimulation ventriculaire à renvoyer en utilisant ledit agencement de câbles d'électrode (11, 12),
**caractérisé en ce que**
ladite évaluation basée sur ledit au moins un critère de stabilité inclut au moins une parmi : l'évaluation si une amplitude de pic d'un pic atrial détecté est supérieure à un premier seuil prédéfini, et l'évaluation si une amplitude de pic d'un pic atrial détecté est inférieure à un second seuil prédéfini.

2. Dispositif de thérapie de resynchronisation cardiaque implantable (1) selon la revendication 1, **caractérisé en ce que** l'unité de commande (100) est configurée pour réaliser ladite routine d'adaptation (S2) si et seulement si le test de détection (S1) est réussi.

3. Dispositif de thérapie de resynchronisation cardiaque implantable (1) selon la revendication 1 ou 2, **caractérisé en ce que** l'unité de commande (100) est configurée pour inhiber la réalisation de la routine d'adaptation (S2) si le test de détection (S1) n'est pas réussi.

4. Dispositif de thérapie de resynchronisation cardiaque implantable (1) selon l'une des revendications précédentes, **caractérisé en ce que** ladite évaluation basée sur ledit au moins un critère de stabilité inclut au moins un parmi : l'évaluation si un intervalle atrial détecté est à l'intérieur d'une première plage prédéfinie, l'évaluation si un changement dans un intervalle atrial détecté sur un nombre prédéfini de cycles cardiaques est à moins d'une première limite prédéfinie, l'évaluation si une amplitude de pic d'un pic atrial détecté est à l'intérieur d'une seconde plage prédéfinie, et l'évaluation si un changement d'une amplitude de pic d'un pic atrial détecté sur un nombre prédéfini de cycles cardiaques est à moins d'une seconde limite prédéfinie.

5. Dispositif de thérapie de resynchronisation cardiaque implantable (1) selon l'une des revendications précédentes, **caractérisé en ce que** l'unité de commande (100) est configurée, au sein de ladite routine d'adaptation (S2), pour mesurer un délai atrioventriculaire intrinsèque en se basant sur un signal de détection atriale et un signal de détection ventriculaire et pour adapter au moins un paramètre en se basant sur un délai atrioventriculaire intrinsèque mesuré.

6. Dispositif de thérapie de resynchronisation cardiaque implantable (1) selon la revendication 5, **caractérisé en ce que** l'unité de commande (100) est configurée pour définir un délai atrioventriculaire stimulé en se basant sur ledit délai atrioventriculaire intrinsèque mesuré et pour générer des signaux de stimulation ventriculaire à renvoyer en utilisant ledit agencement de câbles d'électrode (11, 12) en se basant sur ledit délai atrioventriculaire stimulé.

7. Dispositif de thérapie de resynchronisation cardiaque implantable (1) selon la revendication 5 ou 6, **caractérisé en ce que** l'unité de commande (100) est configurée pour générer des signaux de stimulation ventriculaire soit pour une stimulation bi-ventriculaire soit pour une stimulation dans un seul des ventricules (LV, RV) en se basant sur ledit délai atrioventriculaire intrinsèque mesuré.

8. Dispositif de thérapie de resynchronisation cardiaque implantable (1) selon l'une des revendications précédentes, **caractérisé en ce que** l'unité de commande (100) est configurée, au sein de ladite routine d'adaptation (S2), pour mesurer un premier délai atrioventriculaire intrinsèque en se basant sur un signal de détection atriale et un premier signal de détection ventriculaire détecté dans l'un des ventricules (LV, RV) et un second délai atrioventriculaire intrinsèque en se basant sur un signal de détection atriale et un second signal de détection ventriculaire détecté dans l'autre des ventricules (LV, RV).

9. Dispositif de thérapie de resynchronisation cardiaque implantable (1) selon la revendication 8, **caractérisé en ce que** l'unité de commande (100) est configurée pour adapter ledit au moins un paramètre en se basant sur une comparaison du premier délai atrioventriculaire et du second délai atrioventriculaire.

10. Dispositif de thérapie de resynchronisation cardiaque implantable (1) selon l'une des revendications précédentes, **caractérisé en ce que** l'unité de commande (100) est configurée pour réaliser ledit test de détection (S1) de manière cyclique en employant une longueur de cycle correspondant à un nombre prédéfini de cycles cardiaques.

11. Dispositif de thérapie de resynchronisation cardiaque implantable (1) selon l'une des revendications précédentes, **caractérisé en ce que** le dispositif de thérapie de resynchronisation cardiaque implantable (1) est un dispositif CRT-D, dans lequel le dispositif CRT-D inclut, en plus de la fonction de resynchronisation, une fonction de défibrillation analogue à celle d'un dispositif ICD.

12. Dispositif de thérapie de resynchronisation cardiaque implantable (1) selon l'une des revendications précédentes, **caractérisé en ce que** le dispositif de thérapie de resynchronisation cardiaque implantable (1) est un dispositif CRT-P, dans lequel le dispositif CRT-P inclut, en plus de la fonction de resynchronisation, une fonction de stimulateur cardiaque pour stimuler l'activité cardiaque.
